(19) Europäisches Patentamt / European Patent Office / Office européen des brevets

(11) **EP 3 818 992 A1**

(12) **EUROPEAN PATENT APPLICATION**
published in accordance with Art. 153(4) EPC

(43) Date of publication:
**12.05.2021 Bulletin 2021/19**

(21) Application number: **19831477.5**

(22) Date of filing: **04.07.2019**

(51) Int Cl.:
*A61L 15/64* [(2006.01)]    *A61L 15/26* [(2006.01)]
*A61L 31/06* [(2006.01)]    *A61L 31/14* [(2006.01)]
*A61P 7/04* [(2006.01)]    *D04H 1/435* [(2012.01)]

(86) International application number:
**PCT/JP2019/026551**

(87) International publication number:
**WO 2020/009174 (09.01.2020 Gazette 2020/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.07.2018 JP 2018128274**

(71) Applicant: **Teijin Pharma Limited
Tokyo 100-0013 (JP)**

(72) Inventors:
- **MOTOYOSHI Tetsuya
Tokyo 100-0013 (JP)**
- **YAMAGUCHI Ayuko
Osaka-shi Osaka 530-0005 (JP)**
- **OONO Akitoshi
Tokyo 100-0013 (JP)**
- **MORI Azusa
Tokyo 100-0013 (JP)**

(74) Representative: **Cockerton, Bruce Roger
Carpmaels & Ransford LLP
One Southampton Row
London WC1B 5HA (GB)**

(54) **ENDOSCOPE HEMOSTATIC MATERIAL**

(57) The present invention is an endoscope hemostatic material comprising a sheet-like nonwoven fabric that is formed from a hydrophobic material that has a bulk density of 40 to 110 mg/cm$^3$ and a film thickness of 400 to 1400 $\mu$m, and also an endoscope hemostatic material comprising a sheet-like nonwoven fabric that is formed from a hydrophobic material and that has an area density of 5.0 to 11.0 mg/cm$^3$ and an average fiber diameter of 0.5 to 0.8 $\mu$m, either of which is suitable for treatment of bleeding through an endoscope.

[Fig. 1]

EP 3 818 992 A1

**Description**

[Technical Field]

The present invention relates to a hemostatic material that is suitable for use at the time of endoscopic surgery.

[Background Art]

Sheet-like hemostatic materials such as, for example, SURGICEL (registered trademark) and TachoSil (registered trademark) have been known for treating bleeding at the time of surgery. However, they are usually used by being applied to a bleeding site by hand.

Regarding physical properties of these hemostatic materials, as for the area density (mg/cm$^2$), followings have been recognized: a description that 10 to 30 is preferable (PTL 1), a description of 7 to 20 in the Example (PTL 2), a description that 1.8 to 15 is preferable (PTL 3), a disclosure of an article of 5.5 in the Example (PTL 4), a description that 3 to 20 is preferable (PTL 5), a description that 1 to 30 is preferable (PTL 6), and a disclosure of an article of 52 in the Example (PTL 8).

Also, as for the bulk density (mg/cm$^3$), followings have been recognized: a description that 230 to 1000 is preferable (PTL 3), a disclosure of an article of 45 to 57 in the Example (PTL 4), and a disclosure of an article of 98 to 103 in the Example (PTL 7).

Furthermore, as for the film thickness ($\mu$m), followings have been recognized: a description that 230 to 1000 is preferable (PTL 3), a disclosure of an article of 960 to 1220 in the Example (PTL 4), a description of 50 to 600 (PTL 6), a disclosure of an article of 1100 to 1250 in the Example (PTL 7), and a disclosure of an article of 645 in the Example (PTL 8).

Also, regarding the materials of the hemostatic material mentioned above, followings have been recognized: a lactic acid / glycolic acid copolymer (PTLs 1, 7), polyglycolic acid (PTL 6), cellulose such as oxidized cellulose and carboxymethyl cellulose (PTLs 2, 3, 6, 8), and the like.

Among those patents, PTL 2 disclosed a nonwoven fabric for hemostasis which is designed especially for the treatment of bleeding at the time of surgery performed under a laparoscope, a kind of endoscope, and comprises oxidized regenerated cellulose fibers having a mechanical strength that is strong enough to maintain its structural integrity when the nonwoven fabric is deployed through a laparoscopic trocar.

However, in recent years, the diameter of the trocar used for surgery has been reduced with the increase of low-invasive surgery; this raised problems of the conventional hemostatic materials with the trocar having the reduced diameter, such as difficulty in insertion through the trocar and, even when inserted successfully, difficulty in deployment after the insertion.

[Summary of Invention]

[Technical Problem]

An object of the present invention is to provide a hemostatic material that is suitable for the treatment of bleeding through a trocar that constitutes an endoscopic device (hereinafter, it is also referred to as an "endoscopic trocar") at the time of surgery under the endoscope such as a laparoscope.

[Solution to Problem]

The inventors of the present invention intensively studied to solve the problems mentioned above. Surprisingly, the inventors of the present invention have found a sheet-like nonwoven fabric comprising a hydrophobic material to become a hemostatic material not only undamaged on its structure when deployed through an endoscopic trocar by setting the bulk density and the film thickness each in a specific range, but also having suppressed adhesiveness to non-bleeding site due to its water-absorbability present only when compressed and having excellent rapidness of hemostasis, and further to become a hemostatic material having not only excellent insertability into the endoscopic trocar but also excellent unfoldability by setting the area density and the average fiber diameter in a specific range, and excellent rapidness of hemostasis. As a result, the inventors of the present invention achieved the present invention.

That is, the first of the present invention is an endoscope hemostatic material comprising a sheet-like nonwoven fabric that is formed from a hydrophobic material and has a bulk density of 40 to 110 mg/cm$^3$, and has a film thickness (thickness) of 400 to 1400 $\mu$m (hereinafter, it is referred to as a "hemostatic material in the first invention").

Also, the second of the present invention is an endoscope hemostatic material comprising a sheet-like nonwoven fabric that is formed from a hydrophobic material and has an area density of 5.0 to 11 mg/cm$^2$, and has an average fiber diameter of 0.50 to 8.0 $\mu$m (hereinafter, it is referred to as a "hemostatic material in the second invention").

[Advantageous Effects of Invention]

The hemostatic material in the first invention maintains its structure and its high hemostatic ability is not impaired even when deployed through the endoscopic trocar, in addition, the adhesiveness to non-bleeding site is suppressed, the insertability into the endoscopic trocar is excellent and the unfoldability after insertion is excellent. These properties are extremely beneficial for endoscopic applications.
Also, the hemostatic material in the second invention has excellent insertability into the endoscopic trocar, excellent unfoldability after insertion, and excellent rapidness of hemostasis, and thus is also extremely beneficial for endoscopic applications.

[Brief Description of Drawings]

Fig. 1 shows a dry spinning device as an example of manufacturing equipment for the endoscope hemostatic material in the present invention.
Fig. 2 shows an example of a nozzle that constitutes the dry spinning device as an example of manufacturing equipment for the endoscope hemostatic material in the present invention.

[Description of Embodiments]

The overall shape of the hemostatic material in the first invention and hemostatic material in the second invention is not particularly limited and, include, for example, rectangle one (rectangular parallelepiped if a thickness of film is included). Also, a rectangle includes a square.
The hemostatic material in the first invention is an endoscope hemostatic material having a specific range of a bulk density and film thickness.
As for the bulk density and film thickness, the hemostatic material in the first invention ranges in the bulk density 40 to 110 mg/cm$^3$, and in the film thickness 400 to 1400 $\mu$m, preferably in the bulk density 60 to 100 mg/cm$^3$ and in the film thickness 600 to 1100 $\mu$m, most preferably in the bulk density 60 to 90 mg/cm$^3$ and in the film thickness 900 to 1100 $\mu$m. Although some variation is permitted for such a bulk density and film thickness, it should not be so large and CV is preferably 20% or less when measured at several different points.
Also, the average fiber diameter of the nonwoven fabric of the hemostatic material in the first invention is 0.50 to 8.0 $\mu$m, preferably 0.80 to 6.0 $\mu$m, and more preferably 1.0 to 4.0 $\mu$m. Although some variation is permitted for such an average fiber diameter, it should not be so large, and CV is preferably 50% or less when the fiber diameter is measured at several different points. When the average fiber diameter is outside the range of 0.50 to 8.0 $\mu$m, the insertability and unfoldability in use of the endoscopic trocar with a reduced diameter are poor.
The hemostatic material in the second invention is an endoscope hemostatic material comprising a non-woven fabric having a specific range of the area density and average fiber diameter. The area density of the hemostatic material in the second invention is 5.0 to 11 mg/cm$^2$, and preferably 5.0 to 10 mg/cm$^2$. Although some variation is permitted for such an area density, it should not be so large, and CV is preferably 20% or less when the area density is measured at several different points. When the area density is outside the range of 5.0 to 11 mg/cm$^2$, insertability and unfoldability in use of the endoscopic trocar with a reduced diameter are poor.
The average fiber diameter of the nonwoven fabrics of the hemostatic material in the second invention is 0.50 to 8.0 $\mu$m, preferably the average fiber diameter is 0.80 to 6.0 $\mu$m, and more preferably 1.0 to 4.0 $\mu$m. Although some variation is permitted for such an average fiber diameter, it should not be so large, and CV is preferably 50% or less when the fiber diameter is measured at several different points. When the average fiber diameter is outside the range of 0.5 to 8.0 $\mu$m, insertability and unfoldability in use of of the endoscopic trocar with a reduced diameter are poor.
Also, it is preferable that the hemostatic material in the second invention comprises a nonwoven fabric having the bulk density of 53 to 105 mg/cm$^3$, and the film thickness of 600 to 1500 $\mu$m.
Also, the hemostatic material in the second invention is preferably an endoscope hemostatic material that has a tensile strength in a specific range.
The tensile strength of the hemostatic material in the second invention is preferably 0.60 to 5.0 N, more preferably 0.70 to 4.0 N, and further preferably 0.80 to 3.0 N. When the tensile strength is outside the range of 0.60 to 5.0 N, the insertability and unfoldability in use of the endoscopic trocar with a reduced diameter are poor.
The LD200 value, formation, of a hemostatic material in the second invention ranges 0 to 50, preferably 0 to 45, and more preferably 0 to 40. The LD200 value of the hemostatic material was measured using an image-processing sensor by placing a sample on top of white backlight illumination in a measurement range of 24 mm length $\times$ 18 mm width. The image data obtained was processed within 255 gradations depending on brightness by image processing. At that occasion, pixels were binarized to pixels having brightness of 200 or more and those having brightness less than 200, the pixels having brightness of 200 or more was converted into numerals and designated as LD200, and the formation was calculated using the following formula.

It was binarized by LD200 and was calculated by the following formula.

$$\text{Formation LD200 value} = \text{LDs}/\text{L0} \times 100$$

LDs: LD200 value of the image data of a sample
L0: LD200 value in the state in which a sample was not placed

The endoscope hemostatic material in the present invention includes the hemostatic material that corresponds to the first invention and to the second invention as well.

Raw materials of the hemostatic material in the first invention and the hemostatic material in the second invention are not limited in particular, as long as they are hydrophobic. However, both of the raw materials preferably include a biodegradable polymer, and particularly preferably only a biodegradable polymer. Such biodegradable polymers include aliphatic polyester, and the examples are polylactic acid, polyglycolic acid, lactic acid / glycolic acid copolymer, polycaprolactone, lactic acid / caprolactone copolymer, polyglycerol sebacic acid, polyhydroxyalkanoic acid, polybutylene succinate, and derivatives thereof.

Among those, they are preferably selected from a group consisting of polyglycolic acid, polylactic acid, polycaprolactone, and a copolymer of two kinds or more of monomers that constitute these polymers (any combination of monomers) and mixtures thereof. Most preferable are polylactic acid and a lactic acid / polyglycolic acid copolymer. For example, a stereo complex of poly-L-lactic acid and poly-D-lactic acid may be used.

Furthermore, an ingredient that does not have any adverse effect on the living body and does not disturb the effects of the present invention may be added inside a monofilament that constitutes a nonwoven fabric in the hemostatic material of the present invention or between the monofilaments.

Because the hemostatic material in the first invention and the hemostatic material in the second invention are used at the time of endoscopic surgery such as laparoscopy, those materials must be sterilized. One example of a preferred sterilization method for that purpose is radiation sterilization. Radioactive rays used for this radiation sterilization include, for example, alpha rays, beta rays, gamma rays, neutron beams, electron beams, and X-rays. Among all, a gamma ray and electron beam are preferable, and an electron beam is the most preferable.

The hemostatic material in the first invention and the hemostatic material in the second invention can be used as medical materials when the bleeding is treated through the endoscopic trocar at the time of endoscopic surgery.

One method for using the hemostatic material in the first invention and hemostatic material in the second invention is, for example, folding the hemostatic material, corresponding to the size (diameter) of the endoscopic trocar, within a diameter of the endoscopic trocar, passing it through the rubber stopper of the trocar with forceps, unfolding the folded material with forceps and the like after passing through, applying (i.e., placing) it to the bleeding site, and stanching the bleeding by compressing the hemostatic material with forceps and the like.

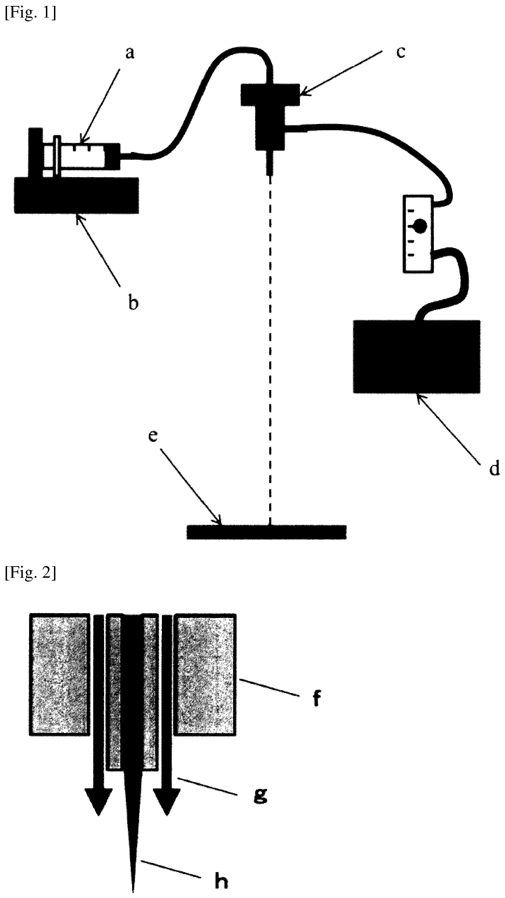

For the method for manufacturing the hemostatic material in the first invention and the hemostatic material in the second invention, depending on the shape thereof, the conventional methods applied specifically to the shape can be used. For example, a dry spinning method is included. The dry spinning method mentioned in the present description is a method such that a nonwoven fabric is manufactured from a solution prepared by dissolving raw materials in a volatile solvent (hereinafter, it is also referred to simply as a "solution") by using airflow that is generated by compressed air and the like. A typical device of the dry spinning method consists of a liquid-delivering pump, such as a syringe pump, which provides propulsive power to send the solution toward a nozzle, a line to send the solution to the nozzle, a nozzle having a shape such that the solution is ejected and airflow is applied to the solution, a compressor that creates compressed air and the like that generate airflows, a line that sends the airflow to the nozzle, and a collector that recovers the solution-spun fibers (Fig. 1). An example of such a nozzle is shown in Fig. 2. The device equipped with a collector transport system, in which the collector moves at a constant speed with respect to the nozzle, makes it possible to obtain a hemostatic material with uniform film thickness and the like, and also to adjust the film thickness and the like by controlling the transportation speed.

[Examples]

Methods for measuring the film thickness, area density, bulk density, average fiber diameter, and for evaluating hemostatic ability and the operability in the endoscopic trocar in the present description are as follows.

A. Film thickness

A high-resolution digital length measuring machine (Mitutoyo Corporation: Instrument name "LITEMATIC VL-50-B") was used. The film thickness of the hemostatic material was determined by measuring at the force of 0.01 N and at 25 mm intervals (one measuring point for 25 mm × 25 mm, four measuring points for 50 mm × 50 mm). When multiple points were measured, the average was defined as the film thickness of the hemostatic material. Also, the minimal measuring force that was available with a measuring machine was applied in the present measurement.

B. Area density and bulk density

The hemostatic material was cut into pieces of 25 mm × 25 mm or 50 mm × 50 mm, and their weight was measured. The bulk density of the hemostatic material was calculated using an area density determined by conversion and the film thickness mentioned above.

C. Average fiber diameter

The surface of the sheet was observed by a scanning electron microscope (SEM), and the width of any 20 fibers in the photos taken by SEM was measured. The average of the measured values was defined as the average fiber diameter.

D. Hemostatic ability

After compression hemostasis was completed, the presence or absence of bleeding from peripheral areas of the hemostatic material was confirmed via an endoscopic camera. That is, intra-abdominal images projected by the endoscope with an integrated video system (VISERA ELITE: Olympus) were observed for three minutes, and it was determined to be "hemostasis" when any bleeding was not recognized.

E. Operability in the endoscopic trocar

Two kinds of the endoscopic trocar, of 12 mm and 5 mm in diameter, were used to evaluate insertability and unfoldability. That is to say, regarding the insertability, when the hemostatic material was inserted, the hemostatic material that was not damaged and was smoothly inserted was indicated as Good, the hemostatic material that was not damaged but was not inserted smoothly due to being caught at the time of insertion was indicated as Poor, and the hemostatic material that was damaged was indicated as Bad. Also, regarding unfoldability, when the hemostatic material inserted into the endoscopic trocar was deployed by using two pairs of forceps, the hemostatic material that was deployed in five seconds or shorter was indicated as Good, the hemostatic material that was deployed in 5 to 10 seconds was indicated as Poor, and the hemostatic material that was deployed in ten seconds or longer was indicated as Bad. Also, as a comprehensive evaluation, for the hemostatic material in which both insertability and unfoldability were judged as Good, or either one of them was judged as Good and the other as Poor, the comprehensive evaluation was indicated as Good. When both of them were Poor or either one was Bad, the comprehensive evaluation of the hemostatic material was indicated as Bad.

F. Formation (LD200 value)

A sample was set on top of a white backlight illuminator (CA-DSW15: KEYENCE CORPORATION) and was measured in a measuring area of 24 mm length × 18 mm width using a high-speed, high-capacity Multi camera image processing sensor (CV-X470F: KEYENCE CORPORATION). The image data obtained was image-processed within 255 gradations depending on brightness. At that occasion, pixels were binarized to pixels having brightness of 200 or more and those having brightness less than 200, the pixels having brightness of 200 or more was converted into numerals and designated as LD200, and the formation was calculated using the following formula.

$$\text{Formation LD200 value} = \text{LDs}/\text{L0} \times 100$$

LDs: LD200 value of the image data of the sample

L0: LD200 value in the state in which a sample was not placed

[Example 1]

Preparation 1 of the hemostatic material

Glycolic acid- / lactic acid copolymer (Purasorb PDLG5010, manufactured by Purac Company) was dissolved in dichloromethane adjusted to be 8% by weight, and a spinning solution of the glycolic acid / lactic acid copolymer was prepared. By using the dry spinning device shown in Fig. 1, a plurality of nonwoven fabrics, as the hemostatic material in the first invention, with different values of the film thickness and the bulk density was obtained according to the dry spinning method, by setting the spinning distance (distance between the tip of a nozzle and a collector) to an appropriate value in the range of 60 to 100 cm, and adjusting the transport speed of the collector to an appropriate value in the range of 55 to 164 mm/min in the dry spinning device equipped with a collector transportation system, under the conditions of the solution delivering speed of 2 mL/min (one hole per nozzle, two holes were used for preparation), and the air flow rate of 100 L/min.

[Example 2]

Water Repellency under the condition without compression

After 9 nonwoven fabric samples prepared in Example 1 were sterilized with an electron beam of 30 kGy, they were cut into pieces having a size of 25 mm × 25 mm to obtain test pieces (N = 3). Five hundred μL of physiological saline was dropped onto a center of each test pieces, and the time until permeation was measured until up to 30 minutes. The results are shown in Table 1.

[Table 1]

| No. | Average film thickness [μm] | Average bulk density [mg/cm$^3$] | Average permeation time [min] |
|-----|------------------------------|-----------------------------------|-------------------------------|
| 1 | 458 | 46 | 17 |
| 2 | 786 | 47 | 30< |
| 3 | 1057 | 49 | 30< |
| 4 | 474 | 65 | 30< |
| 5 | 759 | 73 | 30< |
| 6 | 1058 | 73 | 30< |
| 7 | 467 | 83 | 30< |
| 8 | 737 | 94 | 30< |
| 9 | 1049 | 99 | 30< |

Saline did not permeate even in test piece No. 1 until 17 minutes, which was the quickest permeation among all the test pieces, showing that all nonwoven fabrics tested had high water repellency without compression.

[Example 3]

Water absorbability under the condition with compression

After 9 nonwoven fabric samples prepared in Example 1 were sterilized with an electron beam of 30 kGy, they were cut into pieces having a size of 25 mm × 25 mm to obtain test pieces (N = 3). A wound of 12 mm in diameter and 4 mm in depth was made in the liver of a rabbit (Japanese white, male, 15 weeks of age, KITAYAMA LABES CO., LTD.) (Exudative-hemorrhagic model). The nonwoven fabrics listed in Table 2 were applied to the wound area and compressed with fingers for up to five minutes. The permeation of blood to the nonwoven fabric was visually confirmed.

[Table 2]

| No. | Average film thickness [$\mu$m] | Average bulk density [mg/cm$^3$] |
|---|---|---|
| 1 | 456 | 45 |
| 2 | 733 | 49 |
| 3 | 1034 | 49 |
| 4 | 506 | 66 |
| 5 | 779 | 74 |
| 6 | 1012 | 75 |
| 7 | 483 | 84 |
| 8 | 715 | 92 |
| 9 | 1035 | 99 |

In all the groups, the blood permeation was confirmed at least in part of the nonwoven fabric, and it was found that the nonwoven fabric had water absorbability under the condition with compression.

[Example 4]

Preparation 2 of the hemostatic material

Glycolic acid / lactic acid copolymer (Purasorb PDLG5010, manufactured by Purac Company) was dissolved in dichloromethane adjusted to be 8% by weight, and a spinning solution of the glycolic acid / lactic acid copolymer was prepared. A sheet-like nonwoven fabric was obtained according to the dry spinning method, under the conditions of the spinning distance of 65 cm, the solution delivering speed to 2 mL/min (one hole per nozzle, two holes were used for preparation), the air flow rate of 100 L/min, and the transport speed of the collector of 79 mm/min in the dry spinning device equipped with a collector transportation system. The nonwoven fabric was cut into pieces having a size of 50 mm $\times$ 50 mm and was sterilized with an electron beam of 30 kGy. The nonwoven fabric obtained as the hemostatic material in the first invention had a film thickness of 937 $\mu$m and a bulk density of 86 mg/cm$^3$.

[Example 5]

Evaluation of hemostatic ability under the endoscope (liver)

An endoscopic trocar of 12 mm in diameter was inserted in the abdominal area of a domestic animal pig (female, 2.5 to 4 months old, Aratoyama SPF farm) under anesthesia with inhalation of isoflurane (1% to 3%), and carbon dioxide was sent to bloat the abdominal cavity (pneumoperitoneum). Furthermore, the liver in the intraperitoneal cavity was visualized by an endoscope with an integrated video system (VISERA ELITE: Olympus) after four endoscopic trocars of 12 mm in diameter were inserted. A wound was formed in the liver surface with an electric knife or scissors dedicated to an endoscope, and exudative hemorrhage was induced. The nonwoven fabric (2.4 cm $\times$ 3.0 cm) prepared in Example 4 was folded and inserted in an endoscopic trocar of 12 mm in diameter, then deployed in the peritoneal cavity, and applied (i.e., placed) to the wound site. Compression hemostasis was performed over the hemostatic material using gauze and forceps for three minutes. After compression hemostasis was completed, the observation was carried out for three minutes, and hemostasis was evaluated. In the present experiment, data of six cases were acquired from three animals (2 cases/animal), and a hemostasis rate was calculated. The results are shown in Table 3.
The applied nonwoven fabrics were inserted into the endoscopic trocar without difficulty, and were also deployed without any problem after passing through the endoscopic trocar. During the operation, the structure of the applied nonwoven fabrics did not collapse.

[Table 3]

| Animal number | | 2 | 2 | 3 | 3 | 5 | 5 |
|---|---|---|---|---|---|---|---|
| Number of used fiber formed material | Individual (sheet) | 1 | 1 | 1 | 1 | 1 | 1 |
| | Average (sheet) | 1 | | | | | |

(continued)

| Animal number | | 2 | 2 | 3 | 3 | 5 | 5 |
|---|---|---|---|---|---|---|---|
| Hemostasis evaluation | | Good | Good | Bad | Good | Good | Good |
| Hemostatic rate | Case of hemostasis/ total cases | 5/6 | | | | | |
| | % | 83.3 | | | | | |

Hemostasis was determined in five out of six cases, and the hemostasis rate was 83.3%. Also, the nonwoven fabrics prepared in Example 4 were able to be easily applied to the wound area, and in all six cases, the number of nonwoven fabric piece used until the application was one piece.

[Example 6]

Evaluation of hemostatic ability under the endoscope (spleen)

After the hemostasis experiment in the liver (Example 5) was finished, a wound was formed in the spleen surface with an electric knife or scissors dedicated to the endoscope, and exudative hemorrhage was induced. The nonwoven fabric (2.4 cm × 3.0 cm) prepared in Example 4 was inserted through the trocar of 12 mm in diameter, then deployed in the peritoneal cavity, and applied to the wound site. Compression hemostasis was performed over the hemostatic material using gauze and forceps for three minutes. After compression hemostasis was completed, the observation was carried out for three minutes, and hemostatic was evaluated. In the present experiment, data of six cases were acquired from three animals (2 cases/animal), and a hemostasis rate was calculated. The results are shown in Table 4.

The applied nonwoven fabrics were inserted into the endoscopic trocar without difficulty, and were also deployed without any problem after passing through the endoscopic trocar. During the operation, the structure of the applied nonwoven fabric did not collapse.

[Table 4]

| Animal number | | 2 | 2 | 3 | 3 | 5 | 5 |
|---|---|---|---|---|---|---|---|
| Number of used fiber formed material | Individual (sheet) | 1 | 1 | 1 | 1 | 1 | 1 |
| | Average (sheet) | 1 | | | | | |
| Hemostasis evaluation | | Good | Good | Good | Good | Good | Good |
| Hemostatic rate | Case of hemostasis/ total cases | 6/6 | | | | | |
| | % | 100 | | | | | |

Hemostasis was determined in six out of six cases, and the hemostasis rate was 100%.

Also, the hemostatic materials in the first invention were able to be easily applied to the wound area, and in all six cases, the number of the nonwoven fabric piece used until the application was one piece.

As known from the results in Examples 5 and 6, it was found the hemostatic material in the first invention to be a hemostatic material not only undamaged on its structure when deployed through a endoscopic trocar, but also having suppressed adhesiveness to non-bleeding site, and further having excellent rapidness of hemostasis.

On the other hand, conventional hemostatic materials are in at least any of the following situations: damaged when deployed after passing through the endoscopic trocar, adhering to non-bleeding part of tissues before applied to the bleeding site, making a compression operation itself difficult while applied to the bleeding site, or having insufficient hemostatic ability to stanch bleeding within 3-min compression.

[Example 7]

Preparation 3 of the hemostatic material

Glycolic acid / lactic acid copolymer (Purasorb PDLG5010, a product manufactured by Purac Company) was dissolved in dichloromethane adjusted to be 8% by weight and a spinning solution of glycolic acid / lactic acid copolymer was prepared. A plurality of sheet-like nonwoven fabrics with different values of the film thickness and the bulk density were obtained according to the dry spinning method, by setting the spinning distance (distance between the tip of a nozzle and a collector) to an appropriate value within a range of 50 to 100 cm, and adjusting the transport speed of the collector to an appropriate value within a range of 57 to 1000 mm/min in the dry spinning device equipped with a collector transportation system, under the conditions of the solution delivering speed of 3 mL/min (one hole per nozzle, four holes were used for preparation) and the air flow rate of 130 L/min. A part of the sheet obtained was tucked into a SUS lamina with a gap of 0.8 mm and pressed at 60°C for 15 minutes to obtain a plurality of nonwoven fabrics, as the hemostatic material in the first invention, with different values of the film thickness and the bulk density. These nonwoven fabrics were cut into pieces having a size of 50 mm × 100 mm and sterilized by an electron beam of 30 kGy. Among these nonwoven fabrics, one that corresponded to the hemostatic material in the first invention was designated as No.1, and the ones that did not correspond to were designated as No.2 to No.5 (Table 5).

[Example 8]

Preparation 4 of the hemostatic material

A polylactic acid pellet (REVODE191, manufactured by Daishin Pharma-Chem Co., Ltd.) was used as a raw material, and a sheet-like nonwoven fabric was obtained according to the melt-blow method, by using a nozzle with 301 discharge holes of 0.36 mm in diameter, under the conditions of the polymer discharge rate of 50 g/min, the nozzle temperature of 250°C, and the air flow rate of 4,170 L/min. These nonwoven fabrics, as the hemostatic material in the first invention, were cut into pieces having a size of 50 mm × 100 mm and sterilized by an electron beam of 30 kGy. This nonwoven fabric was designated as No.6, corresponding to the hemostatic material in the first invention (Table 5).

[Example 9]

Water repellency under the condition without compression

The nonwoven fabrics obtained in Examples 7 and 8 were cut into pieces having a size of 10 mm × 10 mm to obtain test pieces. Fifty μL of stored rabbit blood was dropped onto a center of the test piece, and the time until permeation was measured up to 30 minutes (N = 2). The results are shown in Table 5. No. 1 to 5 in Table 5 are the nonwoven fabrics prepared in Example 7, and No.6 is the nonwoven fabric prepared in Example 8.

[Table 5]

| No. | Raw material | Average film thickness [μm] | Average bulk density [mg/cm$^3$] | Permeation time [min] |
|-----|--------------|------------------------------|------------------------------------|------------------------|
| 1 | PLGA | 1030 | 58 | 30< |
| 2 | PLGA | 237 | 42 | 30< |
| 3 | PLGA | 390 | 58 | 30< |
| 4 | PLGA | 995 | 114 | 30< |
| 5 | PLGA | 905 | 163 | 30< |
| 6 | PLA | 549 | 70 | 30< |

The blood did not permeate into any nonwoven fabrics until 30 minutes. It was thus found that the nonwoven fabric possessed high water repellency under the condition without compression.

[Example 10]

Water absorbability under the condition with compression

Pieces of the nonwoven fabric (20 mm × 20 mm) prepared in Examples 7 and 8 were placed on top of Menban (a cotton wool pad) (Hakujuji Co. Ltd., 25 mm × 25 mm) soaked with 3 mL of blood. The pressure was given with 100 g of an acrylic column over the nonwoven fabric for a defined period, and blood permeation into the hemostatic material was visually confirmed (N = 3). The results are shown in Table 6. No. 1 to 5 in Table 6 are nonwoven fabrics prepared in Example 7 and No.6 in Table 6 is the nonwoven fabric prepared in Example 8.

[Table 6]

| No. | Raw material | Average film thickness [μm] | Average bulk density [mg/cm³] | Average blood absorption time [min] |
|---|---|---|---|---|
| 1 | PLGA | 1030 | 58 | 5 |
| 2 | PLGA | 237 | 42 | 0.5> |
| 3 | PLGA | 390 | 58 | 0.5> |
| 4 | PLGA | 995 | 114 | 30< |
| 5 | PLGA | 905 | 163 | 30< |
| 6 | PLA | 549 | 70 | 2.3 |

In any of No.1, No.2, No.3 and No.6, blood permeation was observed within five minutes of the compression, while in No.4 and No.5, the blood did not permeate until 30 minutes of the compression. The water repellent effect under the condition with compression must last 0.5 minutes or longer when the hemostatic material is nipped and applied to a target site with forceps and the like through the endoscopic trocar. In No. 2 and No.3, blood permeated within 0.5 minutes of the compression, thus it was determined that these were not suitable for the operation under the endoscope.

[Example 11]

Preparation 5 of the hemostatic material

Glycolic acid / lactic acid copolymer (Purasorb PDLG5010, manufactured by Purac Company) was dissolved in dichloromethane adjusted to be 8% by weight, and a spinning solution of the glycolic acid / lactic acid copolymer was prepared. By using the dry spinning device shown in Fig. 1, a plurality of sheet-like nonwoven fabrics with different values of the area density and the fiber diameter was obtained according to the dry spinning method, by setting the spinning distance (distance between the tip of a nozzle and a collector) to an appropriate value in a range of 30 to 100 cm, and adjusting the transport speed of the collector to an appropriate value in a range of 55 to 164 mm/min in the dry spinning device equipped with a collector transportation system, under the conditions of the solution delivering speed of 3 mL/min (one hole per nozzle, four holes were used for preparation) and the air flow rate of 130 L/min. The nonwoven fabric obtained was treated at 75°C for 15 minutes, and the film thickness, bulk density, average fiber diameter, and tensile strength were evaluated.

The sheet was cut into pieces having a size of 1 cm × 5 cm and the tensile strength was measured five times with a tensile tester (EZ Test EX) under the conditions of the load cell of 20 N, the distance between chucks of 20 mm, and the speed of 20 mm/min, and then the tensile strength was calculated as their average value.

Among these nonwoven fabrics, the ones that corresponded to the hemostatic material in the second invention were designated as No. 1 to No.4, and the ones that did not correspond to were designated as No.7 to No.9 (Table 7, Table 8).

[Example 12]

Preparation 6 of the hemostatic material

REVODE191 (manufactured by Zhejiang Hisun Biomaterials Co., Ltd.) as a polylactic resin was melted with an extruder, and a sheet-like nonwoven fabric was obtained according to the melt-blow method using a nozzle that has 301 discharge hole of 0.36 mm in diameter under the conditions of the nozzle temperature of 250°C and the air flow rate of 4,170 L/min. On that occasion, a plurality of sheet-like nonwoven fabrics with different values of the area density and the fiber diameter were obtained by regulating the discharge rate and the speed of the belt conveyor. The film thickness, bulk density, average fiber diameter, and tensile strength of the nonwoven fabric obtained were evaluated.

The sheet was cut into pieces having a size of 1 cm × 5 cm and the tensile strength was measured five times with a tensile tester (EZ Test EX) under the conditions of the load cell of 20 N, the distance between chucks of 20 mm, and the speed of 20 mm/min, and then the tensile strength was calculated as their average value.

Among these nonwoven fabrics, the ones that corresponded to the hemostatic material in the second invention were designated as No.5 and No.6, and the ones that did not correspond to it were designated as No. 10 and No. 11 (Table 7, Table 8).

The nonwoven fabrics prepared in Examples 11 and 12 are shown as summarized in Table 7.

[Table 7]

| No. | Polymers | Area density | Average fiber diameter | Film thickness | Bulk density | Tensile strength |
|---|---|---|---|---|---|---|
| | | mg/cm$^2$ | $\mu$m | $\mu$m | mg/cm$^3$ | N |
| 1 | PLGA | 7.4 | 1.7 | 853 | 87 | 1.1 |
| 2 | PLGA | 7.3 | 1.9 | 973 | 75 | 0.9 |
| 3 | PLGA | 7.7 | 1.8 | 1400 | 55 | 0.9 |
| 4 | PLGA | 8.6 | 2.1 | 845 | 102 | 1.1 |
| 5 | PLA | 7.6 | 7.6 | 861 | 88 | 1.2 |
| 6 | PLA | 8.7 | 4.9 | 916 | 95 | 0.9 |
| 7 | PLGA | 2.0 | 1.7 | 427 | 48 | 0.4 |
| 8 | PLGA | 3.9 | 1.9 | 749 | 52 | 0.5 |
| 9 | PLGA | 12.0 | 1.8 | 1064 | 113 | 2.9 |
| 10 | PLA | 3.8 | 8.3 | 549 | 70 | 0.7 |
| 11 | PLA | 14.9 | 8.3 | 752 | 198 | 12.3 |

[Example 13]

Operability study with the endoscopic trocar

After the hemostatic materials prepared in Examples 11 and 12 were sterilized by an electron beam of 30 kGy, they were cut into pieces having a size of 2.4 cm × 3.0 cm. Regarding the nonwoven fabric that was cut, its insertability and unfoldability were evaluated using two trocars having a diameter of 12 mm or 5 mm, and then comprehensive evaluation was carried out. The results are shown as summarized in Table 8. Here, the nonwoven fabrics listed as No. 1 to 11 in Table 8 are identical to the nonwoven fabrics listed with the same numbers in Table 7.

[Table 8]

| No. | Trocar with $\varphi$12 experiment | | | Trocar with $\varphi$5 experiment | | |
|---|---|---|---|---|---|---|
| | Insertability | Unfoldability | Evaluation | Insertability | Unfoldability | Evaluation |
| 1 | Good | Good | Good | Good | Good | Good |
| 2 | Good | Good | Good | Good | Good | Good |
| 3 | Good | Good | Good | Poor | Good | Good |
| 4 | Good | Good | Good | Poor | Good | Good |
| 5 | Good | Good | Good | Good | Good | Good |
| 6 | Good | Good | Good | Poor | Good | Good |
| 7 | Good | Bad | Bad | Bad | Bad | Bad |
| 8 | Good | Poor | Good | Good | Bad | Bad |
| 9 | Good | Good | Good | Bad | Good | Bad |
| 10 | Good | Poor | Good | Bad | Poor | Bad |
| 11 | Good | Poor | Good | Bad | Bad | Bad |

[Example 14]

Preparation 7 of the hemostatic material

Glycolic acid / lactic acid copolymer (Purasorb PDLG5010, manufactured by Purac Company) was dissolved in dichloromethane adjusted to be 8% by weight, and a spinning solution of glycolic acid / lactic acid copolymer was prepared. By using the dry spinning device as shown in Fig. 1, a plurality of sheet-like nonwoven fabrics with different values of the area density and fiber diameter were obtained according to the dry spinning method, by setting the spinning distance (distance between the tip of a nozzle and a collector) to an appropriate value in a range of 30 to 100 cm, and adjusting the transport speed of the collector to an appropriate value in a range of 55 to 164 mm/min in the dry spinning device equipped with a collector transportation system, under the conditions of the solution delivering speed of 2 to 6 mL/min (one hole per nozzle, four holes were used for preparation) and the air flow rate of 130 L/min. The nonwoven fabric obtained was treated at 75°C for 15 minutes, and the film thickness, bulk density and LD200 value were evaluated. The results are shown as summarized in Table 9.

[Table 9]

| No. | Polymers | Area density mg/cm$^2$ | Film thickness $\mu$m | Bulk density mg/cm$^3$ | LD200 value |
|---|---|---|---|---|---|
| 1 | PLGA | 10.5 | 1194 | 88 | 8 |
| 2 | PLGA | 7.0 | 1087 | 64 | 27 |
| 3 | PLGA | 6.8 | 1382 | 49 | 41 |
| 4 | PLGA | 7.3 | 1278 | 57 | 47 |
| 5 | PLGA | 7.0 | 1257 | 56 | 49 |
| 6 | PLGA | 7.5 | 1291 | 58 | 65 |
| 7 | PLGA | 7.7 | 1199 | 64 | 76 |

[Example 15]

Operability study with the endoscopic trocar

The hemostatic material prepared in Example 14 was cut into pieces having a size of 2.4 cm × 3.0 cm. Regarding the nonwoven fabric that was cut, its insertability and unfoldability were evaluated using two endoscopic trocars having a diameter of 12 mm or 5 mm, and then comprehensive evaluation was carried out. The results are shown as summarized in Table 10. Here, the nonwoven fabrics listed as No. 1 to 6 in Table 10 are identical to the nonwoven fabrics listed with the same numbers in Table 9.

[Table 10]

| No. | Experiment with φ12 trocar | | | Experiment with φ5 trocar | | |
|---|---|---|---|---|---|---|
| | Insertability | Unfoldability | Evaluation | Insertability | Unfoldability | Evaluation |
| 1 | Good | Good | Good | Poor | Good | Good |
| 2 | Good | Good | Good | Good | Good | Good |
| 3 | Good | Good | Good | Good | Good | Good |
| 4 | Good | Good | Good | Good | Poor | Good |
| 5 | Good | Good | Good | Good | Poor | Good |
| 6 | Good | Good | Good | Bad | Poor | Bad |
| 7 | Good | Poor | Good | Bad | Bad | Bad |

[Industrial Applicability]

The endoscope hemostatic material in the present invention can be utilized, for example, in the manufacturing industry of medical materials.

[Reference Signs List]

a. Syringe
b. Syringe pump
c. Nozzle
d. Compressor
e. Collector
f. Nozzle
g. Airflow (such as compressed air)
h. Solution

[Citation List]

[Patent Literature]

[PTL 1] Japanese Unexamined Patent Application Publication No. Sho 59-91962
[PTL 2] Japanese Unexamined Patent Application Publication (Translation of PCT Application) No. 2014-504898
[PTL 3] Japanese Unexamined Patent Application Publication No. 2015-70944
[PTL 4] Japanese Unexamined Patent Application Publication No. Hei 8-302553
[PTL 5] Japanese Unexamined Patent Application Publication No. 2004-232160
[PTL 6] Japanese Unexamined Patent Application Publication No. 2015-96081
[PTL 7] Japanese Unexamined Patent Application Publication (Translation of PCT Application) No. 2009-533568
[PTL 8] Japanese Unexamined Patent Application Publication No. 2005-169103

Claims

1. An endoscope hemostatic material comprising a sheet-like nonwoven fabric that is formed from a hydrophobic material and has a bulk density of 40 to 110 mg/cm$^3$ and a film thickness of 400 to 1400 $\mu$m.

2. An endoscope hemostatic material comprising a sheet-like nonwoven fabric that is formed from a hydrophobic material and has an area density of 5.0 to 11.0 mg/cm$^2$ and an average fiber diameter of 0.50 to 8.0 $\mu$m.

3. The endoscope hemostatic material according to claim 2, comprising a nonwoven fabric that has the bulk density of 53 to 105 mg/cm$^3$ and the film thickness of 600 to 1500 $\mu$m.

4. The endoscope hemostatic material according to claim 2 or 3, wherein a tensile strength thereof is 0.60 to 5.0 N.

5. The endoscope hemostatic material according to any one of claims 1 to 4, wherein the hydrophobic material comprises a biodegradable polymer.

6. The endoscope hemostatic material according to any one of claims 1 to 4, wherein the hydrophobic material consists only of a biodegradable polymer.

7. The endoscope hemostatic material according to claim 5 or 6, wherein the biodegradable polymer is an aliphatic polyester.

8. The endoscope hemostatic material according to claim 7, wherein the aliphatic polyester is selected from the group consisting of polyglycolic acid, polylactic acid, polycaprolactone and a copolymer of monomers that constitute these polymers, and a mixture thereof.

[Fig. 1]

[Fig. 2]

## INTERNATIONAL SEARCH REPORT

| International application No. |
| --- |
| PCT/JP2019/026551 |

### A. CLASSIFICATION OF SUBJECT MATTER
Int.Cl. A61L15/64(2006.01)i, A61L15/26(2006.01)i, A61L31/06(2006.01)i, A61L31/14(2006.01)i, A61P7/04(2006.01)i, D04H1/435(2012.01)n

According to International Patent Classification (IPC) or to both national classification and IPC

### B. FIELDS SEARCHED

Minimum documentation searched (classification system followed by classification symbols)
Int.Cl. A61L15/64, A61L15/26, A61L31/06, A61L31/14, A61P7/04, D04H1/435

Documentation searched other than minimum documentation to the extent that such documents are included in the fields searched
| | |
| --- | --- |
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/MEDLINE/EMBASE/BIOSIS (STN), JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| Y | JP 2010-246750 A (TEIJIN LTD.) 04 November 2010, claim 1, paragraphs [0021], [0028], [0044]-[0045], examples 1, 2 (Family: none) | 1-8 |
| Y | JP 2014-504898 A (ETHICON, INC.) 27 February 2014, claims 1, 8, paragraphs [0005], [0032], [0045] & US 2012/0115384 A1, claims 1, 8, paragraphs [0005], [0035], [0046] & WO 2012/064687 A2 & EP 2638194 A2 & CN 103298436 A & KR 10-2013-0101109 A | 1-8 |

☒ Further documents are listed in the continuation of Box C.　　☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 13 September 2019 (13.09.2019) | 01 October 2019 (01.10.2019) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.

PCT/JP2019/026551

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 2013-505109 A (ETHICON, INC.) 14 February 2013 & US 2011/0070288 A1 & WO 2011/037760 A2 & EP 2480260 A2 & CN 102630169 A | 1-8 |
| A | JP 2012-100912 A (GUNZE LIMITED) 31 May 2012 (Family: none) | 1-8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHO5991962 B
- JP 2014504898 PCT
- JP 2015070944 A
- JP HEI8302553 B
- JP 2004232160 A
- JP 2015096081 A
- JP 2009533568 PCT
- JP 2005169103 A